# EUROPEAN PATENT SPECIFICATION

(11) **EP 2 240 123 B1**
(45) Date of publication and mention of the grant of the patent: **23.12.2015**
(21) Application number: 08855527.1
(22) Date of filing: 17.11.2008
(51) Int. Cl.: A61F 2/38

(54) **TOTAL KNEE PROSTHESIS**
KNIEGELENKSTOTALPROTHESE
PROTHÈSE TOTALE DU GENOU

(30) Priority: 27.11.2007 CH 18412007
(43) Date of publication of application: 20.10.2010
(73) Proprietor: Adler Ortho S.r.l., 20032 Cormano (IT)
(72) Inventor: RAGBIR, Sheila, Whim (TT)
(74) Representative: Modiano, Micaela Nadia
(86) International application number: PCT/IB2008/003097
(87) International publication number: WO 2009/068951

(56) References cited:
- EP-A- 0 442 330
- EP-A- 0 546 726
- WO-A-2006/074503
- CA-A1- 2 201 799
- GB-A- 2 296 443
- US-A- 6 013 103

## Description

The present invention relates to the field of prostheses for joints, and more specifically to the part of this field concerned with artificial joints for knees.

As is known, prostheses are used in order to eliminate the pain caused to a joint by a disease and also to restore good mobility of the limb concerned; together with the hip, the knee joint is one of the most important joints in the human body.

The present invention relates to a three-compartment knee prosthesis as defined in the preamble of Claim 1.

In the earlier period of orthopaedics, the use of an artificial joint as a hip prosthesis rapidly became widespread, whereas the use of knee prostheses encountered more serious obstacles initially and took longer to be accepted; the first industrially produced total knee prosthesis only appeared in 1970.

As a result of a series of innovations in the years after 1970, the use of the knee prosthesis became a fully established and reliable procedure with few risks or contra-indications. Total joint replacement has transformed the life of numerous patients, enabling them to remain active with little or no pain and with the restoration of acceptable mobility.

The use of knee prostheses has now reached the same level as the use of hip prostheses in the industrialized countries. The disease which is most commonly responsible for the degeneration of the knee joint is osteoarthritis. Osteoarthritis is a degenerative disease which affects the cartilage, causing lesions in it.

In both the hip and the knee, it is usually the pain that persuades the patient to seek surgical assistance, but whereas in the past the patient could only hope for the elimination of pain, prostheses used at the present time must also meet requirements of attractive appearance, mobility and reliability. It should be emphasized that prostheses are currently fitted to increasingly younger patients who are still active, and will not be satisfied simply by the elimination of pain, but wish to reacquire the functions and appearance that were present before the onset of the disease.

The knee is the anatomic region which joins the thigh and the lower leg, forming the lower limb, and it is a complex joint which has five ligaments (two lateral ligaments, two cruciate ligaments and the patellar ligament), two meniscuses (medial and lateral) and three bone compartments (femoral, tibial and patellar).

When all three bone compartments are prosthetized, the prosthesis is described as "three-compartment"; when the patella is not prosthetized, the prosthesis is described as "two-compartment"; and when only one of the two condyles is prosthetized the prosthesis is described as "single-compartment".

The principal features of a knee prosthesis are the stability and the possibility of a good degree of bending. The importance of stability is evident: when walking, the patient must feel secure without external supports. The importance of bending is not so obvious, but in fact it is just as essential. It should be borne in mind that, if there is not a good degree of bending, it is impossible to stand up from a chair or armchair without using the arms for assistance.

Known prostheses are usually modular; the modularity of a prosthesis makes it possible to choose different sizes for each component and thus assemble the best possible prosthesis to match the characteristics of each individual patient.

The features of the preamble of claim 1 are known from US-A-6013103.

The invention will now be described more fully with reference to the attached drawings which show schematically some preferred embodiments, provided solely as non-limiting examples, since technical or constructional changes can be made at any time without departure from the scope of the present invention as defined by the appended claims.

The object of the prosthesis proposed by the invention is to provide greater mechanical stability and greater flexibility than those of known prostheses, as a result of its structural characteristics, and particularly its design. These objects are achieved by means of the characteristics defined in Claim 1.

In said drawings,

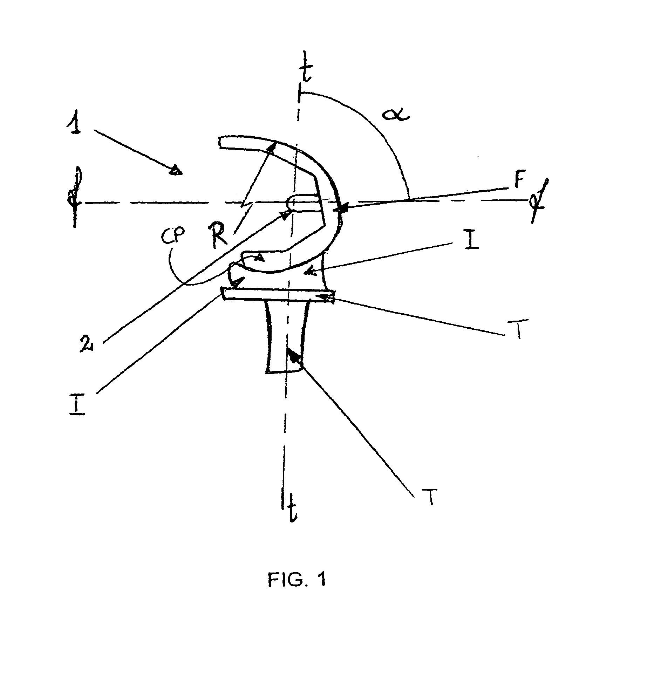

- Figure 1 is a side view of the knee prosthesis, showing the femoral component F and the tibial component T.

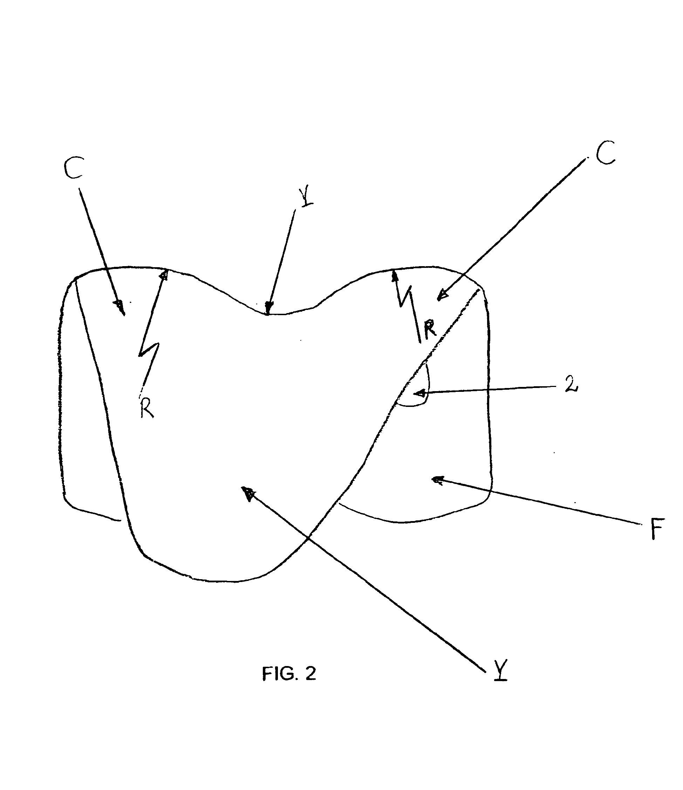

- Figure 2 shows only the femoral component F in front view, said illustration clearly showing the transverse profile of the condyles C linked by a concave surface Y, and also showing part of a fixing element 2.

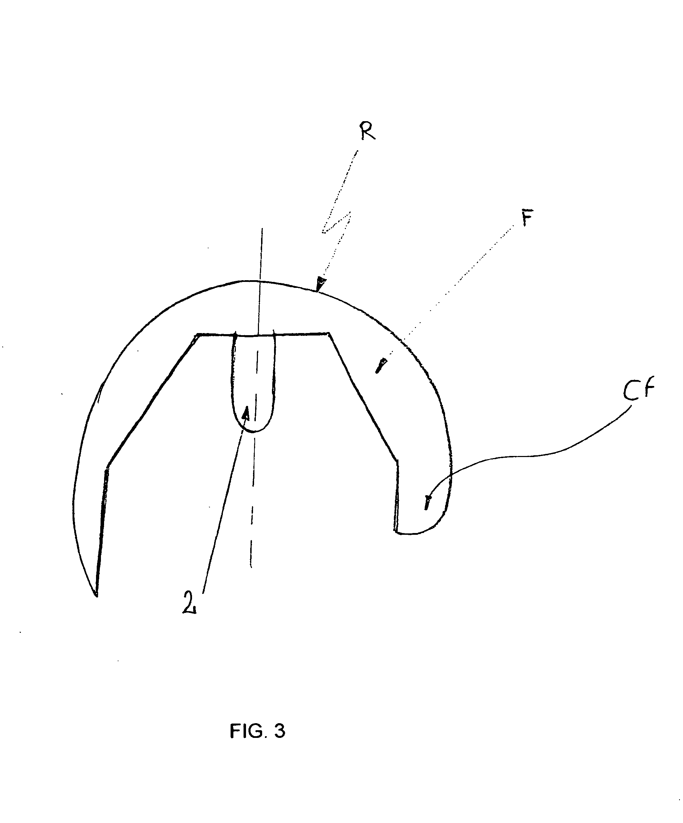

- Figure 3 shows a side view of the femoral component F, the illustration clearly showing the longitudinal profile of the rounded surface, particularly that of the condyles C.

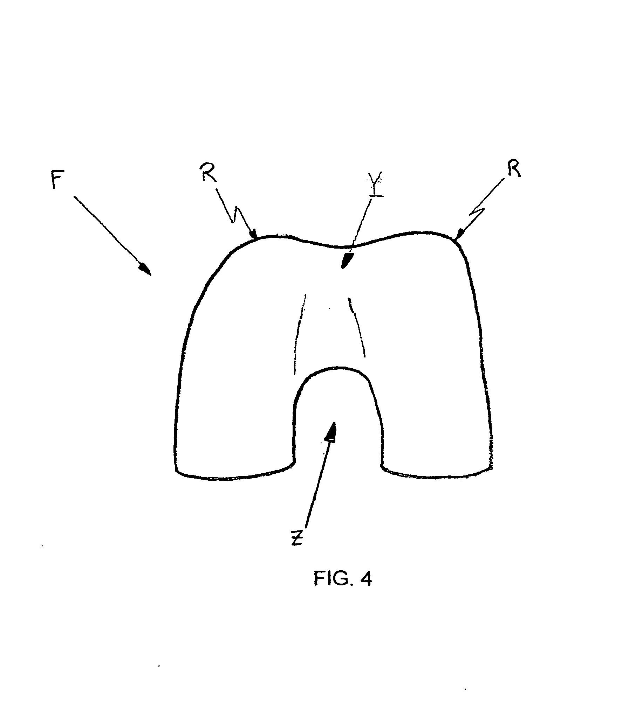

- Figure 4 shows the femoral component F viewed from the rear.

The invention proposes a three-compartment knee prosthesis, comprising a femoral component F which replaces the distal part of the femoral bone, a tibial component T which replaces the proximal part of the tibial bone, a spacer I which is interposed between the two metal components F and T, and finally a patellar component R which replaces the contact surface of the patellar bone.

The femoral component F is made from a CrCoMo (chromium-cobalt-molybdenum) alloy, a hard metal which provides optimal sliding on the upper surface of the spacer I, thus reducing the wear of the polyethylene to a minimum.

The hollow part of the femoral component F which comes into contact with the bone has fixing means (stems) which fix said component to the femoral bone.

The outer part of the femoral component F which comes into contact with the polyethylene spacer has two condyles C, separated from each other to allow space for the posterior cruciate ligament.

Said two condyles C, which are polished to a mirror finish, have the characteristic of having the same radius R both longitudinally and transversely; they are also inserted into the complementary cavities of the polyethylene spacer I interposed between the tibial plate and the femoral component, thus allowing said femoral component F to pivot on the tibial component T.

The femoral component F is thus composed of an inner hollow part with a rough surface providing an optimal grip on the femoral epiphysis, while its outer surface is rounded; in particular, it has two condyles C and a patellar portion.

The tibial component T is also made from a CrCoMo (chromium-cobalt-molybdenum) alloy in the case of a moving plate prosthesis; in a fixed plate prosthesis, however, it is normally made from a titanium alloy in a different shape.

The part of the patellar component that slides on the femoral component is made from high-density polyethylene, while the part which enters the patellar bone can be made from metal.

Said spacer I is interposed between the femoral component F and the tibial component T, this spacer also being made from polyethylene and being free to rotate on the tibial plate T when a moving plate prosthesis is constructed.

The femoral component F also has the following structural characteristics: a constant radius of rotation R of the longitudinal condyle surface starting from the vertical axis t-t remains constant for 95° towards the posterior condyles CP and for a further 70° towards the area of the additional extension.

When the spacer made with the same radius R is used, this provides a maximum contact surface, and consequently maximum stability, between the femoral condyles and the spacer surface, even at maximum extension.

A further characteristic of the femoral component is a special shape of the two posterior condyles, with the bone of the two posterior condyles cut at minus 3 degrees, providing a degree of bending easily exceeding 120°.

## Claims

1. Three-compartment knee prosthesis (1) for replacing the joint between the femur and tibia, comprising:
a femoral component (F) having a patellar portion and two condyles (C), comprising a transverse profile linked by a concave surface (Y),
- a tibial component (T),
- a spacer (I),
said spacer (I) having cavities with longitudinal and transverse profiles having a single curvature (R) for accommodating said two condyles (C),
said two condyles (C) having a constant radius both transversely and longitudinally, which extends over at least 165° longitudinally; said prosthesis being **characterized by** futher comprising a patellar component, wherein
- a part of the patellar component that slides on the femoral component is made from high-density polyethylene,
- said tibial component (T) has a plane on which said spacer (I) rotates, and
- an inner part of the posterior condyles (CP) has a -3° re-entrant profile enabling the terminal parts of the outer parts of the condyles to be shaped so as to allow a degree of bending exceeding 120°.

2. Three-compartment knee prosthesis (1) according to Claim 1, in which the two condyles (C) are linked together by a hollow area (Z) for the passage of the posterior cruciate ligament.

3. Three-compartment knee prosthesis (1) according to any one of the preceding claims, in which the anchoring stems of the femoral component are parallel to the inner surfaces of the posterior condyles (CP).

4. Three-compartment knee prosthesis (1) according to any one of the preceding claims, in which there is a lightening of the medial anterior condyle in said concave area (Y).

## Patentansprüche

1. Knieprothese (1) mit drei Fächern zum Ersetzen des Gelenks zwischen dem Oberschenkelknochen und dem Schienbein, die umfasst:
eine Oberschenkelkomponente (F), die einen Kniescheibenabschnitt und zwei Gelenkköpfe (C) besitzt und ein Querprofil aufweist, das durch eine konkave Oberfläche (Y) verbunden ist,
- eine Schienbeinkomponente (T),
- einen Abstandshalter (I), wobei der Abstandshalter (I) Hohlräume mit Längs- und Querprofilen, die eine einzige Krümmung (R) haben, besitzt, um die zwei Gelenkköpfe (C) aufzunehmen, wobei die zwei Gelenkköpfe (C) sowohl in Quer- als auch in Längsrichtung einen konstanten Radius haben, der sich in Längsrichtung über wenigstens 165° erstreckt;
wobei die Prothese **dadurch gekennzeichnet ist, dass** sie ferner eine Kniescheibenkomponente umfasst, wobei
- ein Teil der Kniescheibenkomponente, der auf der Oberschenkelkomponente gleitet, aus einem hochdichten Polyethylen hergestellt ist,
- die Schienbeinkomponente (T) eine Ebene besitzt, in der sich der Abstandshalter (I) dreht, und
- ein innerer Teil der posterioren Gelenkköpfe (CP) ein -3°-Wiedereintrittsprofil besitzt, das den Endteilen der äußeren Teile der Gelenkköpfe eine Form ermöglicht, um einen Beugungsgrad, der 120° überschreitet, zuzulassen.

2. Knieprothese (1) mit drei Fächern nach Anspruch 1, wobei die zwei Gelenkköpfe (C) miteinander durch einen Hohlbereich (Z) für den Durchgang des posterioren Kreuzbandes verbunden sind.

3. Knieprothese (1) mit drei Fächern nach einem der vorhergehenden Ansprüche, wobei die Verankerungsschafte der Oberschenkelkomponente zu den inneren Oberflächen der posterioren Gelenkköpfe (CP) parallel sind.

4. Knieprothese (1) mit drei Fächern der vorhergehenden Ansprüche, wobei in dem konkaven Bereich (Y) eine Gewichtserleichterung des medialen anterioren Gelenkkopfes vorgesehen ist.

## Revendications

1. Prothèse du genou (1) à trois compartiments pour remplacer l'articulation entre le fémur et le tibia, comprenant :
- un composant fémoral (F) possédant une portion rotulienne et deux condyles (C) comprenant un profil transversal lié par une surface concave (Y) ;
- un composant tibial (T) ;
- un espaceur (I), ledit espaceur possédant des cavités dont les profils longitudinaux et transversaux possèdent une courbure unique (R) pour que viennent s'y loger lesdits deux condyles (C), lesdits deux condyles (C) possédant un rayon constant à la fois en direction transversale et en direction longitudinale qui s'étend sur au moins 165° en direction longitudinale ;
ladite prothèse étant **caractérisée par le fait qu'**elle comprend en outre un composant rotulien,
- une partie du composant rotulien qui glisse sur le composant fémoral étant constituée de polyéthylène haute densité ;
- ledit composant tibial (T) possédant un plan sur lequel tourne ledit espaceur (I) ; et
- la partie interne des condyles postérieurs (CP) possédant un profil rentrant formant un angle de -3° qui permet de configurer les parties terminales des parties externes des condyles de manière telle que l'on peut tolérer un degré de flexion qui est supérieur à 120°.

2. Prothèse du genou (1) à trois compartiments selon la revendication 1, dans laquelle les deux condyles (C) sont reliés l'un à l'autre par une zone creuse (Z) pour le passage du ligament croisé postérieur.

3. Prothèse du genou (1) à trois compartiments selon l'une quelconque des revendications précédentes, dans laquelle les tiges d'ancrage du composant fémoral sont parallèles aux surfaces internes des condyles postérieurs (C).

4. Prothèse du genou (1) à trois compartiments selon l'une quelconque des revendications précédentes, dans laquelle le condyle antérieur médial est allégé dans ladite zone concave (Y).
